# EUROPEAN PATENT APPLICATION

(11) **EP 2 330 122 A1**
(43) Date of publication of application: **08.06.2011**
(21) Application number: 09014931.1
(22) Date of filing: 02.12.2009
(51) Int. Cl.: C07K 14/47

(54) **OFA/iLRP derived modified peptide**

(71) Applicant: Asklepios Kliniken Hamburg Gmbh, 20099 Hamburg (DE)
(72) Inventor: Siegel, Sandra, 21073 Hamburg (DE); Zeis, Matthias, Dr., 22609 Hamburg (DE)
(74) Representative: Glawe, Delfs, Moll

(57) **Abstract**

The present invention relates to a novel modified human oncofoetal antigen immature laminin receptor (OFA/iLR) ― derived peptide comprising the sequence VLIENPADV and its use for diagnostic and therapeutic purposes, specifically in cancer.

## Description

The present invention relates to a novel modified human oncofoetal antigen immature laminin receptor (OFA/iLR)-derived peptide and its use for diagnostic and therapeutic purposes, specifically in cancer. The present invention relates to immunotherapeutic methods, and molecules and cells for use in immunotherapeutic methods. In particular, the present invention relates to the immunotherapy of cancer, in particular several tumor entities including hematological malignancies such as leukemia, multiple myeloma and T cell lymphoma, solid cancers such as breast cancer, colon cancer, lung cancer, kidney cancer, gastric cancer, ovarian cancer, prostate cancer, renal cancer, as well as any cancer that expresses the human oncofoetal antigen immature laminin receptor protein [OFA/iLRP). The present invention furthermore relates to a tumor-associated T-helper cell peptide epitope, alone or in combination with other tumor-associated peptides, that serve as active pharmaceutical ingredients of vaccine compositions which stimulate anti-tumor immune responses. In particular, the present invention relates to one novel modified peptide sequence derived from HLA class I of the human oncofoetal antigen immature laminin receptor protein (OFA/iLRP) which can be used in vaccine or other pharmaceutical compositions for eliciting anti-tumor immune responses.

For the purposes of the present invention, all references as cited herein are incorporated by reference in their entireties.

### BACKGROUND OF THE INVENTION

The process by which the mammalian immune system recognizes and reacts to foreign or alien materials is a complex one. The immune system responds to cancer cells in complicated ways. There are two main types of immune cells that play a significant role in fighting disease: B (or bone marrow-derived) lymphocytes ("B cells) produce antibodies to foreign antigens (which constitutes the part of the immune system known as humoral immunity); and T (or thymus-derived) lymphocytes ("T cells") are involved in cell-mediated immunity. An important facet of the system is the T cell response. Stimulation of an immune response is dependent upon the presence of antigens recognized as foreign by the host immune system. T cells react with foreign antigens via receptors on their surfaces. The T cell receptor is capable of recognizing a particular antigen only when it is associated with a surface marker on an antigen-presenting cell (APC). The surface markers belong to a group of molecules known as the major histocompatibility complex (MHC). The MHC-molecules of the human are also designated as human leukocyte-antigens (HLA). There are two major classes of MHC-molecules, which can be recognized by T cells bearing T cell receptors: MHC-I-molecules, that can be found on most cells having a nucleus that present peptides which result from proteolytic cleavage of endogenous proteins and larger peptides. MHC-II-molecule can be found on professional antigen presenting cells (APC), such as Macrophages, Dendritic Cells, on B cells, on endothelial cells and on altered cells of tumors and tumor stroma which do, under normal circumstances, not express MHC class II-molecules on their cell surfaces, and present either peptides stemming exogenous proteins that are taken up by APCs during the course of endocytosis, or that otherwise enter the MHC class II compartment (MIIC) and are subsequently processed and loaded onto MHC class II complexes. MHC-I-presented peptides are recognized by CD8⁺-positive cytotoxic T lymphocytes, MHC-II-presented peptides are recognized by CD4⁺-helper-T-cells (generally described in Immunobiology by Charles A., Jr. Janeway, Paul Travers, Mark Walport, Mark J. Shlomchik). The APC processes the antigenic protein into shorter peptides called epitopes. If the peptides are presented on class I MHC proteins to CDB⁺ T cells, then the epitopes are usually 8 to 12 residues in length and contain two conserved residues ("anchor") in their sequence that interact with the corresponding binding groove of the MHC-molecule (see 2nd listing as published in Immunogenetics (Rammensee H, Bachmann J, Emmerich NP, Bachor OA, Stevanovic S. SYFPEITHI: database for MHC ligands and peptide motifs. Immunogenetics. 1999 Nov; 50(3-4): 213-9). If the peptides are presented on class II MHC molecules to CD4⁺ T cells, then the epitopes are usually 9 - 30 amino acids in length. Binding of the T cell receptor to the epitope of the antigen on the antigen-presenting cell induces changes in the T cell that triggers (elict) a cell-mediated immune response. This process is dependent on the allele of the MHC-molecule and specific polymorphisms of the amino acid sequence of the peptide. Two signals are primarily responsible for inducing the T cell mediated response to an APC associated with an epitope of an antigen. A first signal results from the binding crosslinking of the T cell receptors with the epitope: MHC protein complex. A second, costimulatory signal is sent by "accessory" membrane molecules on the APC when bound by their receptors on the responding T-cell. Subsequent to the resulting activation of T cells is the secretion of soluble intercellular messengers, known as "cytokines", which regulate the amplitude or intensity and duration of the immune response. Cytokines include the group of biomolecules formerly known as lymphokines, monokines, interleukins and interferons (Essential Immunology, seventh edition, Blackwell Scientific Publications, Oxford, Great Britain, 1991, pp. 140- 150). In this fashion, cytotoxic T cells (CTL) that recognize and are specific to the tumor rejection antigen are stimulated and destroy tumor cells that express the antigen (Cheever et al., Annals N.Y. Acad. Sci. 1993 690:101-112; Rosenberg SA. Shedding light on immunotherapy for cancer. N Engl J Med. 2004 Apr 1;350(14): 1461-3). There are now numerous examples of human CD8⁺ T cells that specifically recognize tumor cells and having therapeutic activity after adoptive transfer, in some cases inducing complete remission. However, despite the potential for T cells to eradicate tumors, it is obvious from the progressive growth of most cancers that many tumors escape recognition by CD8⁺ CTLs *in vivo.* Though a variety of tumors have been found to be immunogenic, stimulation of an effective anti-tumor immune response has been difficult to demonstrate: latest evidences show that immunizations can lead to strong T cell responses against tumor-associated peptides (Speiser DE, Lienard D, Rufer N, Rubio-Godoy V, Rimoldi D, Lejeune F, Krieg AM, Cerottini JC, Romero P. Rapid and strong human CD8+ T cell responses to vaccination with peptide, IFA, and CpG oligodeoxynucleotide-7909. J Clin Invest. 2005 Mar;l 15(3):739-46. Schag K, Schmidt SM, Muller MR, Weinschenk T, Appel S, Week MM, Grunebach F, Stevanovic S, Rammensee HG, Brossart P. Identification of C-met oncogene as a broadly expressed tumor-associated antigen recognized by cytotoxic T-lymphocytes. Clin Cancer Res. 2004 Jun 1;10(1 1):3658-66). The antigens that are recognized by the tumor specific cytotoxic T lymphocytes, that is, their epitopes, can be molecules derived from all protein classes, such as enzymes, receptors, transcription factors, etc. A comprehensive listing of peptides binding to or eluted from MHC class I or class II molecules can be found on www.syfpeithi.org. Furthermore, tumor associated antigens, for example, can also be present in tumor cells as products of mutated genes. Good examples are MHC- class I ligands, which function as T cell epitopes, from K-ras, BCR-abl and mutated p53. Another important class of tumor associated antigens are tissue-specific structures, such as CT ("cancer testis")-antigens that are expressed in different kinds of tumors and in healthy tissues of the testis. Other tumor associated peptides binding to MHC molecules stem from genes, which are expressed in higher copy numbers in cancer cells compared to healthy cells of the same organ or tissue, as well as compared to healthy cells from other tissues. For c-met as an example, see Schag K, Schmidt SM, Muller MR, Weinschenk T, Appel S, Week MM, Grunebach F, Stevanovic S, Rammensee HG, Brossart P. Identification of C-met oncogene as a broadly expressed tumor-associated antigen recognized by cytotoxic T-lymphocytes. Clin Cancer Res. 2004 Jun 1;10(1):3658-66. Other tumor-associated peptides stem from antigens which are in tumor cells retained and not secreted (e.g. proteins from the mucin gene family). Other sources can be aberrant transcripts (frameshift), peptides from junction sites of post-translational protein-protein fusions. A comprehensive listing of tumor associated antigens described in the scientific literature can be found on www.cancerimmunity.org. Although various tumor associated antigens have been identified, much research effort is being expended to identify additional tumor associated antigens. Some groups of tumor associated antigens, also referred to in the art as tumor specific antigens, are tissue specific. Examples include, but are not limited to, tyrosinase for melanoma, PSA and PSMA for prostate cancer and chromosomal cross-overs such as bcr/abl in lymphoma. However, many tumor associated antigens identified occur in multiple tumor types, and some, such as oncogenic proteins and/or tumor suppressor genes (tumor suppressor genes are, for example reviewed for renal cancer in Linehan WM, Walther MM, Zbar B. The genetic basis of cancer of the kidney. J Urol. 2003 Dec; 170(6 Pt 1):2163-72) which actually cause the transformation event, occur in nearly all tumor types. A more general review of genetic causes of human cancer can be found in "The Genetic Basis of Human Cancer" by Bert Vogelstein, Kenneth W. Kinzler, 2002). For example, normal cellular proteins that control cell growth and differentiation, such as p53 (which is an example for a tumor suppressor gene), ras, c-met, myc, pRB, VHL, and HER-2/neu, can accumulate mutations resulting in upregulation of expression of these gene products thereby making them oncogenic (McCartey et al. Cancer Research 1998 15:58 2601-5; Disis et al. Ciba Found. Symp. 1994 187:198-211). These mutant proteins can be the target of a tumor specific immune response in multiple types of cancer. The ideal tumor antigen for use in a vaccine or at which to direct immunotherapy would be one which is present on all tumor types, absent or masked in normal tissues, evolutionarily conserved, and its function required for the malignancy of the tumor cells. Such an immunogen would be less likely to be able to be down regulated or mutated and still have the tumor cells grow and metastasize optimally. The discovery of the existence of tumor associated antigens has now raised the possibility of using a host's immune system to intervene in tumor growth. It has been shown that the CD8⁺ CTL arm of the immune response, alone or in combination with CD4⁺ Th cells, constitutes the primary anti-tumor effector arm of the adaptive immune response. T-helper cells play an important role in orchestrating the effector function of CTLs in antitumor immunity. T-helper cell epitopes that trigger a T-helper cell response of the ThI type support effector functions of CD8⁺ Killer T cells, which include cytotoxic functions directed against tumor cells displaying tumor-associated peptide/MHC complexes on their cell surfaces. In this way tumor-associated T-helper cell peptide epitopes, alone or in combination with other tumor-associated peptides, can serve as active pharmaceutical ingredients of vaccine compositions which stimulate anti-tumor immune responses. Up till now the focus has mainly been on the CTL arm of the immune response. However, it is becoming more and more clear that the CD4 T cell response plays an essential role in tumor rejection, especially in the induction phase or in the extension of a CTL response *in vivo.* Various mechanisms of harnessing both the humoral and cellular arms of the immune system are currently being explored for cancer immunotherapy.

In order for the proteins to be recognized by the cytotoxic T-lymphocytes as tumor-specific antigen, and in order to be used in a therapy, particular prerequisites must be fulfilled. The antigen should be expressed mainly by tumor cells and not by normal healthy tissues or in rather small amounts. It is furthermore desirable, that the respective antigen is not only present in one type of tumor, but also in high concentrations (e.g. copy numbers per cell). Essential is the presence of epitopes in the amino acid sequence of the antigen, since such peptide ("immunogenic peptide") that is derived from a tumor associated antigen should lead to an *in vitro* or *in vivo* T cell response.

It is therefore an object of the present invention, to provide a novel amino acid sequence for such peptide that have the ability to bind to a molecule of the human major histocompatibility complex (MHC) class-I and trigger T cell responses against cells bearing the peptide in conjunction with MHC molecules on their cell surfaces.

According to the present invention, this object is solved by providing a tumor associated modified peptide that is selected from the amino acid sequence of the human oncofetal antigen-immature laminin receptor protein (OFA/iLRP) wherein the peptide has the ability to bind to a molecule of the human major histocompatibility complex (MHC) class-I, comprising but not limited to the HLA allele expressed most frequently in caucasian populations, HLA-A2 (including subtypes of HLA-A2, such as HLA-A*0201).

The oncofoetal antigen-immature laminin receptor protein (OFA/iLRP) is widely expressed in many types of human tumors including hematopoietic malignancies (Rohrer JW, Barsoum AL, Coggin JH Jr. "The development of a new universal tumor rejection antigen expressed on human and rodent cancers for vaccination, prevention of cancer, and anti-tumor therapy". Mod Asp Immunobiol. 2001; 5:191-195; Barsoum AL, Rohrer JW, Coggin JH. "37kDa oncofoetal antigen is an autoimmunogenic homologue of the 37kDa laminin receptor precursor". Cell MoI Biol Lett. 2000; 19: 5535-5542; Castronovo V. "Laminin receptors and laminin-binding proteins during tumor invasion and metastasis". Invasion Metas. 1993; 13:1-30; Coggin JH Jr, Barsoum, AL, Rohrer JW. "Tumors express both unique TSTA and crossprotective 44 kDa oncofetal antigen". Immunol Today. 1998; 19,405-408; Coggin JH Jr, Barsoum AL, Rohrer JW. "37 kilo Dalton oncofetal antigen protein and immature laminin receptor protein are identical, universal T-cell inducing immunogens on primary rodent and human cancers". Anticancer Res. 1999; 19,5535-5542) but is not present in normal adult differentiated tissues.

OFA-iLRP can be specifically recognized by both T and B lymphocytes making it an attractive target molecule for vaccination approaches in several cancer entities. Utilizing dendritic cells (DC) transfected with OFA-iLR-coding RNA, tumor-specific T cell responses against hematopoietic target cells could be generated both in *vitro* and in vivo (Siegel S, Wagner A, Kabelitz D et al. Coggin, JJr., Barsoum, A., Rohrer, J., Schmitz, N., Zeis, M. Induction of cytotoxic T cell responses against the oncofoetal antigen-immature laminin receptor for the treatment of hematological malignancies. Blood 2003 102, 4416-4423). Furthermore, immunogenic OFA/iLRP-specific peptides could be identified which were able to elict a specific T cell response against a variety of malignant hematological cells e.g. chronic lymphocytic leukemia, acute myeloid leukemia etc. (Siegel, S., Wagner, A., Friedrichs, B., Wendeler, A., Wendel, L., Kabelitz, D., Steinman, J., Barsoum, A., Coggin, J., Rohrer, J., Dreger, P., Schmitz, N. & Zeis, M. Identification of HLA-A*0201-presented T-cell epitopes derived from the oncofetal antigen-immature laminin receptor protein for broadly applicable vaccine therapies. J. Immunol. 2006 176(11): 6935-6944; Siegel, S., Friedrichs, B., Budde, AK., Barsoum, A., Coggin, J Jr., Tiemann, M., Kabelitz, D. & Zeis, M. In-vivo detectable antibodies directed against the oncofetal antigen/immature laminin receptor can recognize and control myeloma cells-clinical implications. Leukemia 2008 22(11):2115-8; Siegel, S., Friedrichs, B., Kloess, M., Barsoum, A., Coggin, J., Rohrer J., Jakob, I., Tiemann, M., Heidorn, K., Schulte, C., Kabelitz, D., Steinmann, J., Schmitz, N. & Zeis, M. Humoral immune responses against the immature laminin receptor protein show prognostic significance in patients with chronic lymphocytic leukemia. Journal of Immunology 2008 180(9):6374-84).

US 4,861,710 discloses a clone comprising a recombinant cDNA clone for encoding cell surface receptor for laminin as well as respective probes.

WO 2006/114307 discloses two naturally processed peptide epitopes from the OFA/iLR protein and their medical use fighting malignant cells.

US 6,753,314 describes a purified protein complex comprising a first polypeptide and a second polypeptide, wherein said complex comprises the amino acid sequences of a first polypeptide (SMI1, SEQ ID NO: 359), and a second polypeptide (BAS1, SEQ ID NO: 518), denoted as ProPair 267a-267b.

An important aspect of the present invention relates to the use of the modified peptide of the invention for the preparation of a medicament for the treatment of cancer. A further aspect relates to the use of the composition or the molecule as mentioned above for the preparation of a medicament for the treatment of cancer.

Still further aspects relate independently to a method for treating cancer in a mammal, such as a human, comprising the administration to a patient suffering from the disease an effective amount of the peptide, composition or a molecule of the invention.

### SUMMARY OF THE INVENTION

The inventors have identified a novel modified HLA-A*0201-specific peptide epitope derived from the OFA/iLR protein. This peptide represents a useful tool for both conducting tumor immunological studies and vaccination strategies in OFA/iLRP-expressing malignancies.

The present invention provides a tumor associated modified peptide comprising the sequence according to SEQ ID NO. 1. The novel modified peptide of the present invention has an increased ability to bind to a molecule of the human major histocompatibility complex (MHC.) class-I, in particular to HLA-A*0201, compared to its naturally processed analogue. Further, the peptide of the present invention, when bound to HLA-A*0201, is capable of eliciting the production of a cytotoxic T lymphocyte (CTL), which then recognizes a cell that expresses a polypeptide comprising the given amino acid sequence.

The modified peptides of the present invention may include non-peptide bonds or may be a fusion protein.

The present invention also contemplates pharmaceutical compositions, comprising a modified peptide and a pharmaceutically acceptable carrier. The invention further provides a tumor associated peptide of the invention for use in medicine.

Another embodiment of the present invention provides a cancer vaccine comprising a novel modified peptide of the invention.

The invention further provides the use of a modified peptide of the invention in the manufacture of a medicament for killing target cells in a patient, which target cells expressing a polypeptide comprising an amino acid sequence of the peptide of the present invention, as provided in SEQ ID NO. 1

The present invention further provides activated cytotoxic T lymphocyte (CTL), produced by methods discussed above, which selectively recognize a cell that expresses a polypeptide comprising a peptide of the present invention.

The present invention also provides a T-cell receptor (TCR) that recognizes a cell that expresses a polypeptide comprising a peptide of the present invention, the TCR being obtainable from the cytotoxic T lymphocyte (CTL) described above, or a functionally equivalent molecule to the TCR.

The present invention also provides use of cytotoxic T lymphocytes in the manufacture of a medicament for killing target cells in a patient. The cytotoxic T lymphocytes target cells expressing a polypeptide comprising the peptide of the present invention.

The invention in a further aspect relates to a method of killing target cells in a patient, which target cells express a polypeptide comprising an amino acid sequence as given herein, the method comprising administering to the patient an effective amount of a peptide according to the present invention wherein the amount of said peptide is effective to provoke an anti-target cell immune response in said patient.

The invention in a further aspect relates to a method of killing target cells in a patient which target cells express a polypeptide comprising an amino acid sequence as given according to the present invention, the method comprising the step of obtaining cytotoxic T lymphocytes (CTL) from the patient.

Preferably, the target cells are cancer cells. More preferably, said cancer is leukemia or lymphoma which expresses the polypeptide which comprises an amino acid sequence as given according to the present invention.

It should be understood that the features of the invention as disclosed and described herein can be used not only in the respective combination as indicated but also in a singular fashion without departing from the intended scope of the present invention.

### DETAILED DISCLOSURE OF THE INVENTION

As mentioned above, the HLA system represents the human major histocompatibility (MHC.) system. Generally, MHC systems control a range of characteristics: transplantation antigens, thymus dependent immune responses, certain complement factors and predisposition for certain diseases. More specifically, the MHC codes for three different types of molecules, i. e. Class I, II and III molecules, which determine the more general characteristics of the MHC. Of these molecules, the Class I molecules are so-called HLA-A-molecules that are presented on the surface of most nucleated cells and thrombocytes.

The present invention further relates to one novel modified peptide sequence derived from HLA class I molecules of the oncofoetal antigen-immature laminin receptor protein, which can be used for diagnostic or in vaccine compositions for eliciting anti-tumor immune responses. The modified peptide of the invention is derived from the known (wild-type) sequence of OFA/iLRP, e. g. the sequence disclosed in WO-A-8802407. The term "wild-type" refers to a gene or gene product which has the characteristics of that gene or gene product when isolated from a naturally occurring source. A wild-type gene is what is most frequently observed in a population and is thus arbitrarily designated the "normal" or "wild-type" form of the gene. In contrast, the term "modified" or "mutant" refers to a gene or gene product which displays modifications in sequence and/or functional properties (i.e. altered characteristics) when compared to the wild-type gene or gene product. The modified peptide of the present invention is characterized by its ability to bind to (being restricted to) a particular MHC Class I HLA molecule. The novel peptide sequence have been identified by a new and generally applicable combined approach for the identification of processed HLA class I ligands of defined - e.g. tumor associated - antigens. It is preferred if the peptide of the invention is able to bind to HLA- A2. It is particularly preferred if the peptide bind selectively to HLA-A*0201.

The modified peptide has the sequence VLIENPADV (SEQ ID No. 1). The respective position of the modified peptide in the respective protein is AA₆₆ - AA₇₄. The Accession-numbers of human-OFA/iLR in the Genbank of the "National Centre for Biotechnology Information" of the National Institute of Health (see http: www.ncbi.nlm.nih.gov) are, for example, AACS0652 or AAP35883.

The invention provides a modified peptide, comprising an modified amino acid sequence according to SEQ ID No.1 or a variant thereof provided that the peptide is not the intact human polypeptide from which the amino acid sequence is derived (i.e. oncofoetal antigen-immature laminin receptor protein (OFA/iLRP).

As described herein below, the modified peptide that form the basis of the present invention has been identified as being presented by MHC class I bearing cells. Thus, this particular peptide as well as other peptides containing the sequence (i.e. derived peptides) will most likely elicit a specific T-cell response, although the extent to which such response will be induced might vary from peptide to peptide. Differences, for example, could be caused due to mutations in said peptide. The persons of skill in the present art are well aware of methods that can be applied in order to determine the extent to which a response is induced by an individual peptide, in particular with reference to the examples herein and the respective literature.

Thus, the inventors identified one HLA-A*0201-specific T cell epitope derived from the OFA/iLR-protein able to induce specific T cell reactivity against human tumor cells, including but not limited to various hematological malignancies.

Preferably, a peptide according to the present invention consists essentially of an amino acid sequence according to SEQ ID No. 1 or a variant thereof. "Consisting essentially" of shall mean that a peptide according to the present invention, in addition to the sequence according to SEQ ID No. 1 or a variant thereof, could contain additional N-and/or C-terminally located stretches of amino acids that are not necessarily forming part of the peptide that functions as core sequence of the peptide comprising the binding motif and as an immunogenic T-helper epitope. Nevertheless, these stretches can be important in order to provide for an efficient introduction of the peptide according to the present invention into the cells.

By a "variant" of the given amino acid sequence the inventors mean that the side chains of, for example, one or two of the amino acid residues are altered (for example by replacing them with the side chain of another naturally occurring amino acid residue or some other side chain) such that the peptide is still able to bind to an HLA molecule in substantially the same way as a peptide consisting of the given amino acid sequence. For example, a peptide may be modified so that it at least maintains, if not improves, the ability to interact with and bind a suitable MHC molecule, such as HLA-A, and so that it at least maintains, if not improves, the ability to generate activated CTL which can recognize and kill cells which express a polypeptide that contains an amino acid sequence as defined in the aspects of the invention. As can derived from the database as described in the following, certain positions of HLA-A binding peptides are typically anchor residues forming a core sequence fitting to the binding motif of the HLA binding groove.

Those amino acid residues that are not essential to interact with the T cell receptor can be modified by replacement with another amino acid whose incorporation does not substantially effect T cell reactivity and does not eliminate binding to the relevant MHC. Thus, apart from the proviso given, the peptide of the invention may be any peptide (by which term the inventors include oligopeptide or polypeptide) which includes the amino acid sequences or a portion or variant thereof as given.

Typically, the peptide of the invention is one which, if expressed in an antigen presenting cell, may be processed so that a fragment is produced which is able to bind to an appropriate MHC molecule and may be presented by a suitable cell and elicit a suitable T cell response. It will be appreciated that a fragment produced from the peptide may also be a peptide of the invention. Conveniently, the peptide of the invention contains a portion which includes the given amino acid sequence or a portion or variant thereof and a further portion which confers some desirable property. For example, the further portion may include a further T cell epitope (whether or not derived from the same polypeptide as the first T cell epitope-containing portion) or it may include a carrier protein or peptide. Thus, in one embodiment the peptide of the invention is a truncated human protein or a fusion protein of a protein fragment and another polypeptide portion provided that the human portion includes one or more inventive amino acid sequences.

In a particularly preferred embodiment, the peptide of the invention includes the modified amino acid sequence of the invention and at least one further T cell epitope wherein the further T cell epitope is able to facilitate the production of a T cell response directed at the type of tumor that expresses a tumor-associated antigen. Thus, the peptides of the invention include so-called "beads on a string" polypeptides which can also be used as vaccines.

By "aberrantly expressed" the inventors include the meaning that the polypeptide is overexpressed compared to normal levels of expression or that the gene is silent in the tissue from which the tumor is derived but in the tumor it is expressed. By "overexpressed" the inventors mean that the polypeptide is present at a level at least 1.2 x that present in normal tissue; preferably at least 2 x and more preferably at least 5 x or 10 x the level present in normal tissue.

A further aspect of the invention provides a method of killing target cells in a patient which target cells express a polypeptide comprising an amino acid sequence of the invention, the method comprising administering to the patient an effective amount of a peptide according to the invention, or an effective amount of a polynucleotide or an expression vector encoding a said peptide, wherein the amount of said peptide or amount of said polynucleotide or expression vector is effective to provoke an anti-target cell immune response in said patient. The target cell is typically a tumor or cancer cell, in particular a leukemia or lymphoma cell. The term "expression vector" as used herein refers to DNA sequences containing a desired coding sequence and appropriate DNA sequences necessary for the expression of the operably linked coding sequence in a particular host organism or host cell. DNA sequences necessary for expression in procaryotes include a promoter, optionally an operator sequence, a ribosome binding site and possibly other sequences. Eukaryotic cells are known to utilize promoters, polyadenylation signals and enhancers. The terms "in operable combination", "in operable order" and "operably linked" as used herein refer to the linkage of nucleic acid sequences in such a manner that a nucleic acid molecule capable of directing the transcription of a given gene and/or the synthesis of a desired protein molecule is produced. The term also refers to the linkage of amino acid sequences in such a manner so that a functional protein is produced. As used herein, the term "regulatory element" refers to a genetic element which controls some aspect of the expression of nucleic acid sequences. For example, a promoter is a regulatory element which facilitates the initiation of transcription of an operably linked coding region. Other regulatory elements are splicing signals, polyadenylation signals, termination signals etc..Transcriptional control signals in eucaryotes comprise "promoter" and "enhancer" elements. Promoters and enhancers consist of short arrays of DNA sequences that interact specifically with cellular proteins involved in transcription [Maniatis T. et al., Science 236:1237 (1987)]. Promoter and enhancer elements have been isolated from a variety of eukaryotic sources including genes in yeast, insect and mammalian cells and viruses (analogous control elements, i.e., promoters, are also found in procaryotes). The selection of a particular promoter and enhancer depends on what cell type is to be used to express the protein of interest. Some eukaryotic promoters and enhancers have a broad host range while others are functional in a limited subset of cell types [for review see Voss S.D. et al., Trends Biochem. Sci., 11:287 (1986) and Maniatis. T. et al., supra (1987)].

A still further aspect of the present invention provides the use of a peptide according to the invention in the manufacture of a medicament for killing target cells in a patient which target cells express a polypeptide comprising an amino acid sequence of the invention.

It will be appreciated that, with respect to the methods of killing target cells in a patient, it is particularly preferred that the target cells are cancer cells, more preferably leukemia or lymphoma cancer cells.

It is particularly preferred if the patients who are treated by the methods of the invention have the HLA-A2 type. Thus, in a preferred embodiment the HLA haplotype of the patient is determined prior to treatment. HLA haplotyping may be carried out using any suitable method; such methods are well known in the art.

The invention includes the use of the modified peptide of the invention for active in vivo vaccination. In a preferred embodiment, the vaccine of the present invention is administered to a host either alone or in combination with another cancer therapy to inhibit or suppress the formation of tumors.

It will be appreciated from the following that in some applications the modified peptide of the invention may be used directly (i.e. they are not produced by expression of a polynucleotide in a patient's cell or in a cell given to a patient); in such applications it is preferred that the peptide has fewer than 100 or 50 residues. A preferred peptide of the present invention exhibits an overall length of between 9 and 30 amino acids.

The peptide for use in a cancer vaccine may be any suitable peptide. In particular, it may be a suitable 9-mer peptide or a suitable 7-mer or 8-mer or 10-mer or 11-mer peptide or 12-mer. Longer peptides may also be suitable, but 9-mer or 10-mer peptides are preferred.

In another aspect of the present invention, a combination with other peptides, for example MHC class II specific peptides can be used. The person of skill will be able to select preferred combinations of immunogenic peptides by testing, for example, the generation of T cells in vitro as well as their efficiency and overall presence, the proliferation, affinity and expansion of certain T cells for certain peptides, and the functionality of the T cells, e.g. by analyzing the production of IFN-y or GranzymeB. Usually, the most efficient peptides are then combined as a vaccine for the purposes as described above.

A further aspect of the invention therefore provides a vaccine effective against cancer, or cancer or tumour cells, comprising an effective amount of a peptide according to the invention. Most preferred is a vaccine comprising a (synthetic) peptide or peptides (i.e. either alone or in combinations of 1, 2, 3, 4, 5 or 6 or even more peptides).

Polynucleotide-mediated immunization therapy of cancer is described in Conry et al (1996) Seminars in Oncology 23,135-147; Condon et al (1996) Nature Medicine 2,1122-1127; Gong et al (1997) Nature Medicine 3,558-561; Zhai et al (1996) J. Immunol. 156,700-710; Graham et al (1996) Int J. Cancer 65,664-670; and Burchell et al (1996) pp 309-313 In: Breast Cancer, Advances in biology and therapeutics, Calvo et al (eds), John Libbey Eurotext, all of which are incorporated herein by reference.

The invention in a further aspect relates to a pharmaceutical composition, that contains said modified peptide according to the invention. This composition is used for parenteral administration, such as subcutaneous, intradermal, intramuscular or oral administration. Doses of between 1 and 500 mg of peptide may be given. For this, the peptide is dissolved or suspended in a pharmaceutically acceptable, preferably aqueous carrier. The peptide may be substantially pure or an addition, the composition can contain excipients, such as buffers, binding agents, blasting agents, diluents, flavors, lubricants, etc. The peptide can also be administered together with immune stimulating substances, such as cytokines. An extensive listing of excipients that can be used in such a composition, can be, for example, taken from A. Kibbe, Handbook of Pharmaceutical Excipients, 3. Ed., 2000, American Pharmaceutical Association and pharmaceutical press. Preferably, the peptide as active pharmaceutical component is given in combination with an adjuvant, such as, for example, IL-2, IL-12, GM-CSF, incomplete Freund's adjuvant, complete Freund's adjuvant or liposomal formulations. The most preferred adjuvants can be found in, for example, Brinkman JA, Fausch SC, Weber JS, Kast WM. Peptide-based vaccines for cancer immunotherapy. Expert Opin Biol Ther. 2004 Feb;4(2): 181-98. The peptide may also be conjugated to a suitable carrier such as keyhole limpet hemocyanin (KLH) or mannan (see WO 95/18145 and Longenecker et al (1993) Ann. NY Acad. Sci. 690,276-291). The peptide vaccine may be administered without adjuvant. The peptide may also be tagged, or be a fusion protein, or be a hybrid molecule. The peptide which sequence is given in the present invention is expected to stimulate CD8⁺ CTL. However, stimulation is more efficient in the presence of help provided by CD4⁺ T cells. Thus, the fusion partner or sections of a hybrid molecule suitably provide epitopes which stimulate CD4⁺ T cells. CD4⁺ stimulating epitopes are well known in the art and include those identified in tetanus toxoid. The composition can be used for a prevention, prophylaxis and/or therapy of tumoros diseases.

Vaccination results in CTL responses stimulated by professional antigen presenting cells; once CTL are primed, there may be an advantage in enhancing MHC expression in tumor cells.

The activated CTL express a T cell receptor (TCR) which is involved in recognizing cells which express the polypeptide. It is useful if the cDNA encoding the TCR is cloned from the activated CTL and transferred into a further CTL for expression.

In vivo, the target cells for the CD8⁺ CTL according to the present invention can be cells of the tumor, leukemia or lymphoma (which express-MHC class I) and/or stromal cells surrounding the tumor (tumor cells) (which sometimes also express MHC class I).

A still further aspect of the invention provides a method of killing target cells in a patient which target cells express a polypeptide comprising an amino acid sequence of the invention, the method comprising the steps of (1) obtaining CTL from the patient; (2) introducing into said cells a polynucleotide encoding a TCR, or a functionally equivalent molecule, as defined above; and (3) introducing the cells produced in step (2) into the patient.

The pharmaceutical preparation, containing the peptide of the present invention is administered to a patient that suffers from a tumorous disease that is associated with the respective peptide or antigen. Particular diseases to be treated are malignancies expressing OFA/iLRP, such as leukemias (e.g. CLL) myelomas (e.g. MM) or T cell lymphomas, solid cancers (e.g. breast cancer, colon cancer, lung cancer, kidney cancer, gastric cancer, ovarian cancer, prostate cancer, renal cancer) as well as any cancer that expresses the human oncofoetal antigen immature laminin receptor protein (OFA/iLRP). By this, a CTL-specific immune response can be triggered.

The peptide according to the invention can also be used as diagnostic reagent. Using the peptide it can be analyzed, whether in a CTL-population CTLs are present that are specifically directed against a peptide or are induced by a therapy. Furthermore, the increase of precursor T cells can be tested with this peptide that have a reactivity against the defined peptide. Furthermore, the peptide can be used as marker in order to monitor the progression of the disease of a tumor that expresses said antigen of which the peptide is derived from.

In another preferred embodiment of the present invention, the peptide is used for the production of an antibody. Polyclonal antibodies can be obtained in a standard fashion by immunization of animals via injection of the peptide and subsequent purification of the immune globulin. Monoclonal antibodies can be produced according to standard protocols such as described, for example, in Methods Enzymol. (1986), 121, Hybridoma technology and monoclonal antibodies.

Finally, the vaccine can be dependent from the specific type of cancer that the patient to be treated is suffering from as well as the status of the disease, earlier treatment regimens, the immune status of the patient, and, of course, the HLA-haplotype of the patient.

### DESCRIPTION OF THE EXPERIMENTAL OF THE INVENTION

A peptide was synthesized which was predicted by the computer programs PAProC (http://www.unituebingen.de/uni/kxi/) and SYFPEITHI (http://www.syfpeithi.de) to bind to the HLA-A*0201 molecule. The inventors of the present invention found that the modified peptide (referred to as "iLR-ASK-A2"; SEQ ID No. 1) having an amino acid sequence in which the second amino acid "Ala" of the known amino acid sequence Val-Ala-Ile-Glu-Asn-Pro-Ala-Asp-Val (SEQ ID No. 2) is changed to "Leu", namely Val-Leu-Ile-Glu-Asn-Pro-Ala-Asp-Val, has a higher binding affinity for the HLA-A*0201-molecule. Furthermore, a higher capability to induce cytotoxic T lymphocytes (CTL) has been observed. Amino acid substitutions can be introduced at anchor positions, but not at TCR contact residues, to increase peptide binding to the HLA class I molecule. Thus, the present invention provides a modified peptide consisting of 9-30 amino acids and comprising the following amino acid sequence: Val-Leu-Ile-Glu-Asn-Pro-Ala-Asp-Val (SEQ ID No. 1). This peptide is preferably a polypeptide consisting of 9-12 amino acids and comprising the amino acid sequence indicated in Sequence ID No. 1 and, more preferably, a peptide consisting of the amino acid sequence indicated in Sequence ID No. 1.

In an *in vitro* reconstitution assay using the TAP (transporter associated with antigen processing)-deficient T2 cell line, the peptide showed strong binding affinity to HLA-A* 0201 if compared with the naturally processed peptide (Sequence ID No. 2).

A significant feature of the modified peptide of the invention is its capability to recognize or elicit INF-y-/GrB-producing responder T cells, i. e. cytotoxic T cells (CTLs) that specifically recognize the particular peptide in a PBMC population or tumor cells of a cancer patient (target cells).

This activity is readily determined by subjecting PBMC or tumor cells from a patient to an ELISPOT assay (as described in Berke, Z. , Andersen, M. H. , Pedersen, M. , Fugger, L., Zeuthen, J., and Haurum, J. S. Peptides spanning the junctional region of both the abl/bcr and the bcr/abl fusion proteins bind common HLA class I molecules. Leukemia, 14: 419-426,2000.) Prior to the assay, it would be advantageous to stimulate the PBMC population or the tumor cells to be assayed by contacting the cells with the peptide to be tested. Preferably, the peptide is capable of eliciting or recognizing INF-y-/GrB-producing T cells at a frequency of at least 1 per 10⁴ PBMC as determined by an ELISPOT assay as used herein. More preferably the frequency is at least 5 per 10⁴ PBMC, most preferably at least 10 per 10⁴ PBMC, such as at least 50 or 100 per 10⁴ PBMC.

The ELISPOT assay represents a strong tool to monitor OFA/iLR peptide specific T cell responses. Such direct evidence is provided herein, as it was that OFA/iLRP reactive cells isolated by means of HLA/peptide complexes possess the functional capacity of lysing target cells. Accordingly the modified peptide of the invention is capable of eliciting INF-y-/GrB-producing cells in a PBMC population of a patient having a cancer disease where OFA/iLRP is expressed including a haematopoietic malignancy such as chronic lymphatic leukemia, multiple myeloma and malignant lymphoma.

Specifically, the modified peptide of the invention is able to elicit an immune response in the form of T cell having cytotoxic effect against OFA/iLRP expressing cells of a cancer and the TAP-deficient T2 cell line loaded with the appropriate iLR-ASK-A2 peptide. Specific CTL lines elicited strong cytolytic activity against allogenic OFA/iLRP expressing HLA-A*0201* CLL cells but spared HLA-A2-negative / OFA/iLRP-positive targets and HLA-A2-positive / OFA/iLRP-negative targets. Antibody blocking experiments revealed an MHC-class I-restricted killing induced by peptide specific CD8⁺ T lymphocytes. According to the low ability to bind the HLA-A2-molecule as descripted by T2 binding assay, no cytotoxicity could be obtained by stimulation of patients PBMC with the naturally processed iLR peptide (SEQ ID No. 2).

### SUMMARY OF THE EXPERIMENTAL RESULTS OF THE INVENTION

Previously data have shown that OFA/iLR-specific regulatory CD8⁺ T cell clones secreting Interleukin-10 can be identified both in mice bearing an OFA/iLR⁺ tumor (Rohrer JW, Rohrer SD, Barsoum A, Coggin JH Jr. Differential recognition of murine tumor- associated oncofoetal transplantation antigen and individually specific tumor transplantation antigens by syngeneic cloned Balb/c and RFM mouse T cells. J Immunol. 1994; 152: 745- 764) and in patients with advanced breast carcinomas (Rohrer JW, Barsoum AL, Dyess DL et al. Human breast carcinoma patients develop clonable oncofoetal antigen-specific effector and regulatory T lymphocytes. J Immunol. 1999; 162: 6880-6892. 11. Rohrer JW, Coggin JH Jr. CD8+ T cell clones inhibit antitumor T cell function by secreting IL-IO. J. Immunol. 1995; 155: 5719). Su et al. (Su Z et al. Immunological and Clinical responses in metastatic renal cancer patients vaccinated with tumor RNA-transfected dendritic cells. Cancer Res. 2003; 63:2127-2133) reported T cells reactive against MHC class I-restricted epitopes from OFA/iLR from patients with metastatic renal cancer. In this study, the authors also report an unexpectedly low mortality of patients having received therapeutic vaccinations with autologous dendritic cells, which had been transfected with RNA encoding OFA/iLR and other putative tumor antigens. However, the number, HLA-restriction and chemical identity of epitopes from OFA/iLR was not disclosed by Su et al. Hδlt1 et al. (Holtl L et al. Immunotherapy of metastatic renal cell carcinoma with tumor lysate-pulsed autologous dendritic cells. Clin. Cancer Res. 2002; 8:3369-3376) delivered further evidence that OFA/iLR may be very useful for cancer immunotherapy based on therapeutic vaccines stimulating specific cellular immune responses. The authors found that 5/6 patients with metastatic renal cell carcinoma (RCC), who had been vaccinated with autologous dendritic cells loaded with autologous or allogeneic tumor cell lysates, featured enhanced immune responses against OFA/iLR. Again, no epitopes from OFA/iLR were disclosed by Holtl et al. Interestingly, the patients with the strongest immune responses against OFA/iLR had a complete and a partial clinical response.

In summary, the inventors identified a novel modified HLA-A*0201-specific peptide epitope derived from the OFA/iLR protein. This peptide represents a useful tool for both conducting tumor immunological studies and vaccination strategies in OFA/iLRP- expressing malignancies.

The invention in a further aspect relates to a method of killing target cells in a patient which target cells express a polypeptide comprising an amino acid sequence as given herein, the method comprising administering to the patient an effective amount of a peptide according to the present invention wherein the amount of said peptide is effective to provoke an anti-target cell immune response in said patient.

Preferably, the target cells are cancer cells. More preferably, said cancer is leukemia or lymphoma which expresses the polypeptide which comprises an amino acid sequence as given according to the present invention.

It should be understood that the features of the invention as disclosed and described herein can be used not only in the respective combination as indicated but also in a singular fashion without departing from the intended scope of the present invention.

### EXPERIMENTAL DESCRIPTION OF THE INVENTION

The invention will now be described in more detail by reference to the following Figures, the Sequence listing, and the Examples. The following examples are provided for illustrative purposes only and are not intended to limit the invention.
Table 1 shows T-cell epitope containing modified peptide SEQ ID No 1 (iLR-ASK-A2) and naturally processed SEQ ID No 2 that are presented by MHC class I according to the present invention.
Figure 1A shows the binding of the Influenza matrix protein FluM1 to the HLA-A2-molecule of the TAP-deficient T2 cell line as a positive control in a T2 binding assay.
Figure 1B shows the strenghtened binding ability of the modified peptide iLR-ASK-A2 to bind to the HLA-A*0201 compared to the binding ability of the naturally processed peptide (SEQ ID No 2) determined by an T2 binding assay.
Figure 2 shows the capability of the modified peptide iLR-ASK-A2 of elicting INF-y-producing T cells determined by an ELISPOT assay.
Figure 3 shows the capability of the modified peptide iLR-ASK-A2 of elicting GranzymeB-producing T cells determined by an ELISPOT assay.
Figure 4 shows the lysis of peptide-pulsed TAP (transporter associated with antigen processing)-deficient T2 target cells by iLR-ASK-A2-specific CTL.
Figure 5 shows that CTL specific for HLA-A*02/iLR-ASK-A2 kill tumor cells from HLA-A2-positive CLL patients but spared HLA-A2-negative CLL cells.
Figure 6 shows the blocking of target cell lysis by antibodies recognizing MHC class I or TCR for iLR-ASK-A2.

### Abbreviations used throughout the present application:

Ab: Antibody
Ag: Antigen
APC: antigen presenting cell
CD: Cluster of Differentiation cpm: counts per minute
DC: Dendritic Cell
HLA: Human Leukocyte Antigen
IFN: Interferon
IL: Interleukin
MHC: Major Histocompatibility Complex
PBMC: Peripheral Blood Mononuclear Cells

### Cell line, tumor samples and peripheral blood mononuclear cells [PBMC):

TAP-deficient T2 cell line used in this study were obtained from American Type Culture Collection (Manassas, VA, USA). PBMC and tumor samples were collected from patients with chronic lymphocytic leukemia (CLL), multiple myeloma (MM) and T cell lymphoma respectively, after informed consent and approval by the institutional review board.

### Antibodies:

Antibody against human HLA-A,B,C: clone W6/32 from BioLegend, Cat.-Nr. 311412.

Antibody against TCR: clone BMA031 from Beckman Coulter, Part.-Nr. IM 1466.

Antibody against HMFG-I _{:} clone 1.10.F3 from Beckman Coulter, Part.-Nr. IM 0271.

### Peptides:

The peptides FIuMl₅₈-₆₆ (GILGFVFTL, positive control in T2 binding assay), HIV-PoI₄₇₆₋₈₄ (ILKEPVHGV, negative control in ELISPOT assay) msurv33 (LYLKNYRIA, murine survivin peptide epitope specific for H2^{d}, negative control in T2 binding assays and ⁵¹Chromium release assay); native iLR₆₆₋₇₄ (VAIENPADV, naturally processed OFA/iLR peptide), iLR-ASK-A2₆₆-₇₄ (VLIENPADV, modified OFA/iLR peptide) were purchased from peptides & elephants (Berlin, Germany) provided with more than 90% purity and were analyzed by high-performance liquid chromatography and mass spectrometry.

### T2-binding assay:

The T2 whole cell-binding assay was performed using a protocol adopted from Casati et al. (Casati C, Dalerba P, Rivoltini L et al. The apoptosis inhibitor protein survivin induces tumor- specific CD8+ and CD4+ T cells in colorectal cancer patients. Cancer Res. 2003; 63, 4507- 4515). The influenza matrix protein served as a positive control.

### Generation of human CTL:

For the induction of OFA/iLR peptide-specific CTL obtained from patients with CLL CDS⁺ T cells were separated from PBMC using immunomagnetic beads (MACS^{®}, Miltenyi, Bergisch-Gladbach, Germany) and co-cultured with autologous irradiated OFA/iLR peptide-pulsed CD8⁺/CD4⁺-depleted PBMC. CTL were weekly restimulated with autologous irradiated peptide-pulsed CD8⁺ICD4⁺-depleted PBMC in the presence of IL-2 (20IU/ml; CellConcepts, Weisskirch, Germany) and IL-7 (10ng/ml; PeproTech, Hamburg, Germany). After at least four weekly restimulations, CTL reactivity was determined in a conventional 4h ⁵¹Chromium release assay. Antibody blocking experiments were performed as previously described (Zeis M, Siegel S, Schmitz M et al. Induction of cytotoxic T lymphocytes against hematologic neoplasms by survivin RNA-transfected dendritic cells. J Immunol. 2003; 170, 5391-5397).

### ELISPOT assay:

To determine the frequency of OFA/iLR peptide-specific T cells in patients, PBMC form patients with CLL, MM and T-NHL were stimulated with the iLR-ASK-A2 peptide in the presence of IL-2 and IL-7 in the same concentration used for the stimulation of CTL as mentioned above for at least 10 days with one restimulation at day 7. ELISPOT-assays were performed as described elsewhere (Siegel, S., Wagner, A., Friedrichs, B., Wendeler, A., Wendel, L., Kabelitz, D., Steinman, J., Barsoum, A., Coggin, J., Rohrer, J., Dreger, P., Schmitz, N. & Zeis, M. Identification of HLA-A*0201-presented T-cell epitopes derived from the oncofetal antigen-immature laminin receptor protein for broadly applicable vaccine therapies. J. Immunol. 2006; 176(11): 6935-6944-) using a monoclonal anti-human-Interferon-y-/GranzymeB-antibody (MabTech, Nacka, Sweden).

To demonstrate the low potential of the natural processed iLR₆₆₋₇₄ peptide to induce a cytotocix T cell answer, PBMC from patients with CLL, MM and T-NHL were stimulated with the native iLR₆₆-₇₄ peptide in the presence of IL-2 and IL-7 in the same concentration used for the stimulation of CTL as mentioned above for at least 10 days with one restimulation at day 7. ELISPOT-assays were performed as described elsewhere (Siegel, S., Wagner, A., Friedrichs, B., Wendeler, A., Wendel, L., Kabelitz, D., Steinman, J., Barsoum, A., Coggin, J., Rohrer, J., Dreger, P., Schmitz, N. & Zeis, M. Identification of HLA-A*0201-presented T-cell epitopes derived from the oncofetal antigen-immature laminin receptor protein for broadly applicable vaccine therapies. J. Immunol. 2006; 176(11): 6935-6944.) using a monoclonal anti-human-Interferon-y-antibody (MabTech, Nacka, Sweden).

### Table 1: Peptides and tumor-associated T-helper cell peptide epitopes as identified in the present Invention

### Name Sequence SEQ ID No.:

1. iLR-ASK-A2₆₆-₇₄ VLIENPADV SEQ ID No. 1
2. native iLR₆₆₋₇₄ VALENPADV SEQ ID No. 2
3. FIuMI₅₈-₆₆ GILGFVETL SEQ ID No. 3 positive control in T2 binding assay
4. HIV-PoI₄₇₆₋₄₈₄ ILKEPVHGV SEQ ID No. 4 negative control in ELISPOT assay
5. msurv33 LYLKNYRIA SEQ ID No. 5 murine survivin peptide epitope specific for H2^{d}, negative control in T2 binding assays

### EXPERIMENTAL RESULTS OF THE INVENTION

### T2-binding assay:

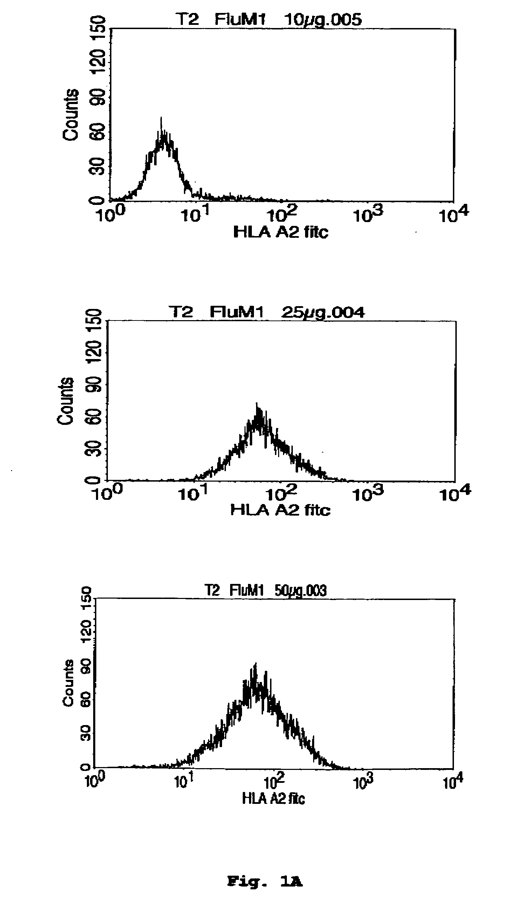

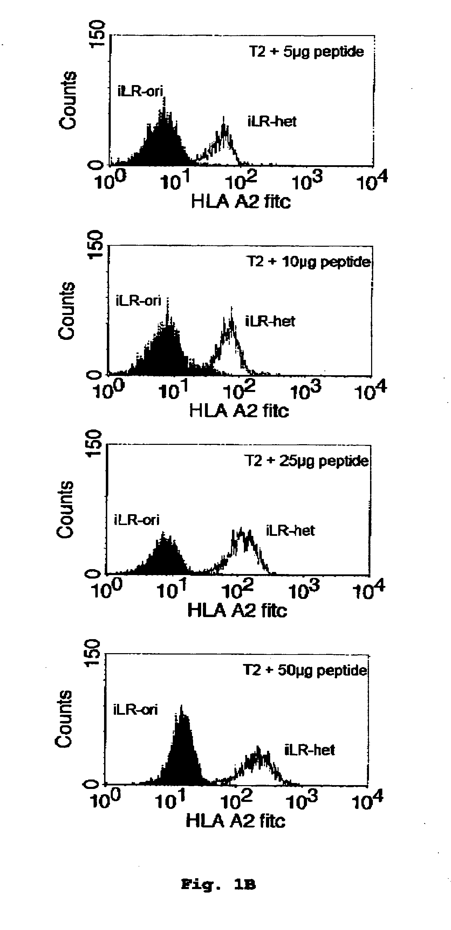

T2-binding assay revealed a strenghtened binding of the iLR-ASK-A2 peptide (SEQ ID No.1) to the HLA-A2-molecule compared to the naturally processed iLR peptide (SEQ ID No. 2) by loading the TAP-deficient T2 cell line with different amonts of peptide determined by flow cytometry (Fig. 1A/B).

### In summary:

1. iLR-ASK-A2 is able to bind the HLA-A*02-molecule
2. iLR-ASK-A2 has an increased ability to bind the HLA-A*02-molecule compared to the naturally processes iLR peptide.

### ELISPOT assays:

In all ELISPOT analyses, the numbers given for IFN-_{Y}-/GrB-positive spots are per 10⁵ PBMC. deducting of background. Prior to analysis, T cells from patients had been kept in culture for ten days in the presence of Interleukin-2 (IL-2; 20 units/ml), Interleukin-7 (IL-7; 10ng/ml) and peptide (10 microgram/ 10⁶ PBMC/ml) .

Of all patient tumor samples probed (e.g. CLL, MM and T-NHL), 1/22 (4.54%) have significant (> 20) CTL responses against the natural processed iLR₆₆-₇₄ peptide in IFN-_{Y} ELISPOT analysis.

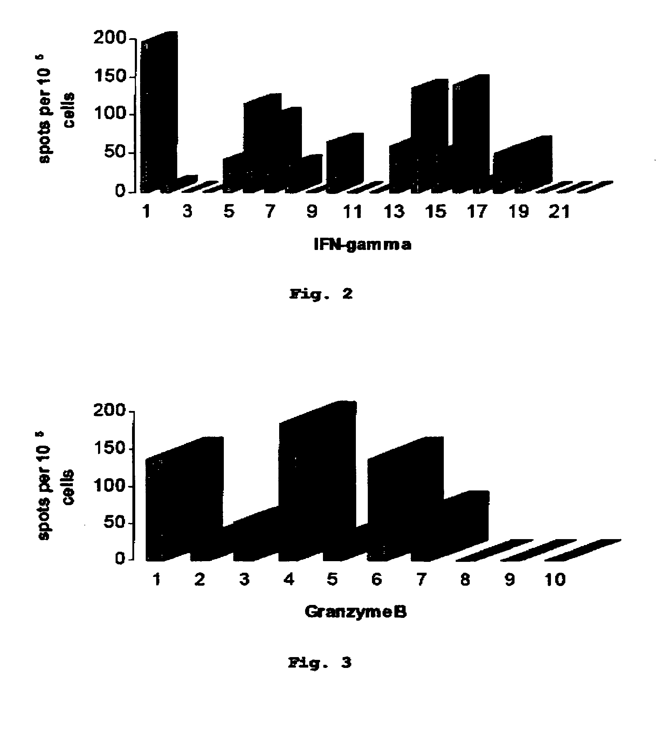

Of all patient tumor samples probed (e.g. CLL, MM and T-NHL), 14/22 (63,63%) have significant (> 20) CTL responses against iLR-ASK-A2 peptide in IFN-_{Y} ELISPOT analysis (Fig. 2).

Of all patient tumor samples probed (e.g. CLL, MM and T-NHL) 7/10 (70%) have significant (> 20) CTL responses against iLR-ASK-A2 peptide in GrB ELISPOT analysis (Fig. 3).

In summary, the potential of peptide iLR-ASK-A2 from OFA/iLRP to evoke cellular immune responses against cancer cells, specifically from leukemias and lymphomas, becomes clearly visible. The T cells specific for said peptide display effector functions (secretion of IFN-_{Y}/GranzymeB).

### Cytotoxicity assay:

### Target: T2

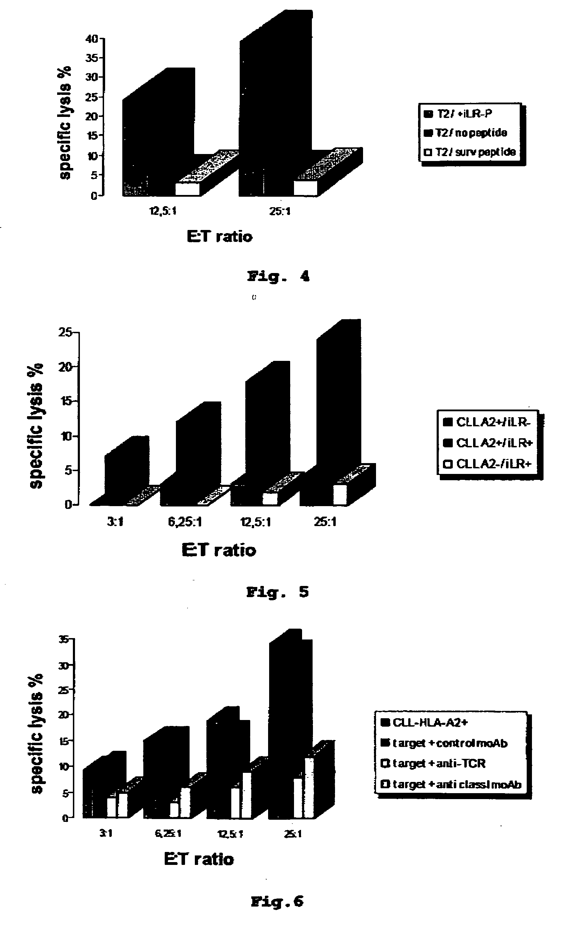

T2 cells are deficient for expression of TAP, the "Transporter associated with Antigen Processing", which is responsible for shuttling short peptides from the cytoplasm to the endoplasmatic reticulum (ER), where peptides are loaded onto empty MHC class I molecules. In consequence, MHC class I molecules of T2 cells remain empty, if no peptides are added (external loading). Thus, T2 cells expressing empty HLA-A*02 molecules on the cell surface are optimal targets for peptide-specific killing by HLA-A* 02-restricted T cells. In this experiment , T cells specific for iLR-ASK-A2 were tested on T2 cells pulsed with the appropriate peptide VLIENPADV, with no peptide or the irrelevant murSurv peptide (SEQ ID No. 5). Results determined by standard ⁵¹Chromium release assay: the T cells recognized only T2 cells pulsed with the iLR-ASK-A2 peptide. T2 cells pulsed with no peptide or the irrelevant murSurv peptide were not recognized (Fig. 4).

### In summary:

1. iLR-ASK-a2 is capable of elicting OFA/iLRP-specific cytotoxic T-lymphocytes in A*02-positive CLL patients
2. iLR-ASK-A2 peptide is recognized in an HLA-restricted fashion.
3. The restriction is allele-specific (specific for A*02).

### Target: CLL,

T cells specific for HLA-A* 02-restricted iLR-ASK-a2 peptide were tested in a standard ⁵¹Chromium release assay on tumor cells from CLL patients. Cells from A*02 ― positive/OFA/iLRP-positive patient were recognized by the iLR-ASK-A2-specific CTL while A*02-negative/OFA/iLRP-positive as well as A*02-positive/OFA/iLRP-negative patients were not recognized in any case (Fig. 5).

### In summary:

1. iLR-ASK-A2 is capable of elicting OFA/iLRP-specific cytotoxic T-lymphocytes in A*02-positive CLL patients
2. iLR-ASK-A2 peptide is recognized in an HLA-restricted fashion.
3. The restriction is allele-specific (specific for A*02).
4. OFA/iLRP-specific cytotoxic T lymphocytes are able to kill OFA/iLRP expressing allogenic target cells.

### Specificity analysis:

Figure 6 shows antibody blocking experiments to further characterize the specificity of the T cell response. Prior to ⁵¹Cr-release experiments at different E:T ratio, blocking mAbs were incubated with HLA-A*0201― positive/OFA/iLRP-positive CLL cells at mAb concentrations as indicated by the manufacturer. The blocking experiments indicate that the recognition of iLR-ASK-A2 is mediated by cells bearing T-cell receptors (TCR), recognizing their target in the context of MHC class I (HLA class I). Control mAbs specific for irrelevant, HMFG-I did not have an effect on the recognition of CLL cells by the effector T cells. PBS was used as a negative control in these blocking experiments.

### In summary, this experiment confirm that

1. iLR-ASK-A2 is capable of elicting OFA/iLRP-specific cytotoxic T-lymphocytes in A*02-positive CLL patients
2. iLR-ASK-A2 peptide is recognized in an HLA-restricted fashion.
3. The restriction is allele-specific (specific for A*02).
4. OFA/iLRP-specific cytotoxic T lymphocytes are able to kill OFA/iLRP expressing allogenic target cells.
5. The interaction between targets and effector cells can be specifically inhibited with mAbs against MHC class I or TCR.

In summary, the inventive modified peptide iLR-ASK-A2 from the OFA/iLR protein is a candidate for developing peptide-based therapeutic vaccines for cancer patients in general.

## Claims

1. A peptide comprising sequence ID No. 1 (VLIENPADV).

2. The peptide according to claim 1, wherein said peptide exhibits an overall length of 9 to 30 amino acid residues, preferably 9 to 20 amino acid residues, further preferably 9, 10, 11, 12 or 13 amino acid residues, further preferably 9 or 10 amino acid residues, further preferably said peptide is consisting of sequence ID No. 1.

3. The peptide according to any of the preceding claims, having the ability to bind to a molecule of the major histocompatibility complex (MHC) class I, preferably restricted by a MHC- class I HLA-A molecule, further preferably selected from the group consisting of HLA-A2, in particular HLA-A*0201.

4. The peptide according to claim 3, wherein said peptide-bound is capable of eliciting the production of cytotoxic T lymphocyte (CTL) which recognizes human oncofoetal antigen immature laminin receptor protein (OFA/iLRP) expressing cells.

5. The peptide according to any of the preceding claims, wherein the peptide is a fusion protein.

6. A nucleic acid, encoding a peptide according to any of the preceding claims, wherein said nucleic acid is preferably selected from the group consisting of DNA, cDNA, PNA, CNA, RNA or combinations thereof.

7. An expression vector capable of expressing a nucleic acid according to claim 6.

8. An isolated host cell comprising a nucleic acid according to claim 6 or an expression vector according to claim 7, wherein said host cell is not an embryonic stem-cell.

9. An antibody recognizing the peptide according to any of claims 1-5, wherein said antibody is preferably a monoclonal antibody specific for peptide sequence ID No. 1 (VLIENPADV).

10. A composition comprising a) the peptide according to claims 1 to 5, a nucleic acid according to claim 6, an expression vector according to claim 7, or an antibody according to claim 9 and optionally b) a pharmaceutically acceptable carrier.

11. The composition of claim 10 for use as a medicament, preferably a medicament for killing OFA/iLRP expressing target cells.

12. The composition of claim 10 for the treatment of cancer, preferably for the treatment of cancer selected from the group consisting of: hematological malignancies such as leukemia, multiple myeloma and T cell lymphoma lymphoma, solid cancers such as breast cancer, colon cancer, lung cancer, kidney cancer, gastric cancer, ovarian cancer, prostate cancer, renal cancer, further preferably from the group consisting of haematopoietic malignancy including chronic lymphatic leukemia, multiple myeloma and T cell lymphoma.

13. A kit for diagnosis of the presence of OFA/iLRP reactive T cells in a blood sample or in tumor tissue, the kit comprising a peptide according to any one of claims 1-5 or a composition of claim 10.

14. An *in-vitro* method for producing activated CTL, the method comprising contacting in-vitro with antigen loaded human class I MHC molecules, preferably restricted by a MHC class I HLA-A molecule, further preferably selected from the group consisting of HLA-A2, in particular HLA-A*0201, expressed on the surface of a suitable antigen-presenting cell for a period of time sufficient to activate, in an antigen specific manner, said CTL, wherein the antigen is a peptide according to any of claims 1 to 5.

15. The method of claim 14, wherein the antigen-presenting cell comprises an expression vector according to claim 7.

16. A method of producing a peptide according to any one of claims 1 to 5 comprising the steps of culturing the host cell according to claim 8 and isolating the peptide from the host cell or its culture medium.


SEQUENCE LISTING

<110>  Asklepios Kliniken Hamburg GmbH

<120>  OFA/iLRP derived modified peptide

<130>  ASKP001PEP

<160>  5

<170>  PatentIn version 3.3

<210>  1
<211>  9
<212>  PRT
<213>  artificial

<220>
<223>  mutation A2L

<400>  1

Val Leu Ile Glu Asn Pro Ala Asp Val
1               5


<210>  2
<211>  9
<212>  PRT
<213>  human

<400>  2

Val Ala Ile Glu Asn Pro Ala Asp Val
1               5


<210>  3
<211>  9
<212>  PRT
<213>  human

<400>  3

Gly Ile Leu Gly Phe Val Phe Thr Leu
1               5


<210>  4
<211>  9
<212>  PRT
<213>  HIV

<400>  4

Ile Leu Lys Glu Pro Val His Gly Val
1               5


<210>  5
<211>  9
<212>  PRT
<213>  murine

<400>  5

Leu Tyr Leu Lys Asn Tyr Arg Ile Ala
1               5